# EUROPEAN PATENT APPLICATION

(11) **EP 1 955 998 A1**
(43) Date of publication of application: **13.08.2008**
(21) Application number: 07101856.8
(22) Date of filing: 07.02.2007
(51) Int. Cl.: C07C 281/16, C07D 209/14

(54) **New addition salt of N-amino-N'-pentylguanidine, the process for its preparation and use thereof for obtaining tegaserod**

(71) Applicant: Chemo Ibérica, S.A., 08028 Barcelona (ES)
(72) Inventor: Marras, Giovanni, 28100, Novara (Novara) (IT)
(74) Representative: Barlocci, Anna

(57) **Abstract**

The present invention refers to a new addition salt of N-amino-N'-pentylguanidine, particularly to the oxalate addition salt of N-amino-N'-pentylguanidine. The process for preparing said oxalate addition salt comprises the steps of: a) reacting the N-amino-N'-pentylguanidine, as a free base or in a salt form, with oxalic acid in a polar medium; and b) recovering the product in a solid form.

This addition salt is useful for obtaining the tegaserod free base with a better purity and yield.

## Description

The present invention relates to a new addition salt of N-amino-N'-pentylguanidine, particularly to the oxalate addition salt of N-amino-N'-pentylguanidine, and to its use for obtaining tegaserod free base and salts thereof.

### BACKGROUND ART

Tegaserod maleate is a selective serotonin 5HT4-receptor partial agonist used in the treatment of irritable bowel syndrome. Its international nonpropietary name is 3-(5-methoxy-1 H-indole-3-yl-methylene)-N-pentylcarbazimidamide monomaleate, and corresponds to formula (I): 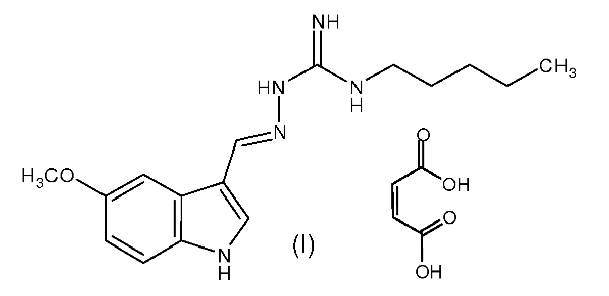 Tegaserod was disclosed in the U.S. Patent No. 5.510.353 A. The patent also discloses the preparation of tegaserod by reacting aminoguanidine hydrochloride with n-pentylamine to give N-pentyl-N-aminoguanidine hydrochloride, which in turn is condensed with 5-methoxy-1 H-indole-3-carboxaldehyde in a protic solvent in the presence of an inorganic or organic acid to give tegaserod base.

The PCT application WO 2005/105740 describes a process for preparing tegaserod comprising the steps of: a) reacting N-amino-N'-pentylguanidine hydroiodide (APG-HI) with 5-methoxy-1 H-indole-3-carbaldehyde (5-MICHO) in water and/or in an organic medium under acidic or basic conditions to obtain tegaserod; b) recovering crude tegaserod as free base; and c) converting tegaserod free base in a corresponding pharmaceutically acceptable salt.

### SUMMARY OF THE INVENTION

The problem to be solved by the present invention is the provision of an alternative process for the preparation of tegaserod as a free base or a salt form.

The solution is based on a new addition salt of N-amino-N'-pentylguanidine (APG), particularly the oxalate addition salt of N-amino-N'-pentylguanidine (hereinafter also referred as "APG-Hox"). When the addition salt of the present invention is used as the starting material for the synthesis of tegaserod (either as a free base or a salt thereof), it can be obtained with higher purity and a better yield.

In WO 2005/105740, the starting material for preparing the tegaserod free base is N-amino-N'-pentylguanidine hydroiodide. This product is an oil and when it is used at industrial scale it gives rise to problems such as its store and manage. Furthermore, as APG-Hl is an iodide salt, the oxidation of iodine can occur, generating iodine forms which can give rise to the coloration of the end product. Consequently, when N-amino-N'-pentylguanidine hydroiodide is used as the starting material for the synthesis of tegaserod free base the purity and yield are low, on one hand because the starting material is not stable enough, suffering from oxidation and then, a minor amount of the end product is obtained compared to the one expected; and on the other hand because the resulting product (i.e. tegaserod) can be coloured, being necessary additional steps for its ulterior purification, which decreases even more the final yield.

It has been found that the oxalate addition salt of the N-amino-N'-pentylguanidine is a more convenient starting material for the synthesis of tegaserod free base. This is due to the fact that this addition salt is obtained as a solid (and not as an oil) and therefore it can be easily purified by crystallization. In this way, controlling the purity of the APG-Hox as the starting material (performing it by cheap techniques, such as crystallization), the purity of tegaserod free base or a salt thereof is also controlled: if APG-Hox is highly pure, the final product (either the free base or salt thereof) is obtained with high purity. The main consequence is that no additional purification steps of the final compound are needed.

Furthermore, since the APG-Hox is a white solid, problems associated with coloration of the final product are avoided and, on the other hand, owing to its stability it is easily transportable.

Thus, in a first aspect the present invention relates to an oxalate addition salt of N-amino-N'-pentylguanidine.

In a second aspect the present invention relates to a process for preparing the oxalate addition salt according to the first aspect of the invention which comprises the steps of: a) reacting the N-amino-N'-pentylguanidine, as a free base or in a salt form, with oxalic acid in a polar medium; and b)recovering the product in a solid form.

In a third aspect the present invention relates to a process for preparing tegaserod free base which comprises the steps of: i) condensing the oxalate addition salt of N-amino-N'-pentylguanidine with 5-Methoxy-1 H-indole-3-carbaldehyde in an organic medium; and ii) recovering tegaserod as free base.

Throughout the description and claims the word "comprise" and variations of the word, such as "comprising", is not intended to exclude other technical features, additives, components, or steps. The content of the application from which priority is claimed, as well as the contents of the abstracts of the priority application and the present application, are incorporated herein as reference. Additional objects, advantages and features of the invention will become apparent to those skilled in the art upon examination of the description or may be learned by practice of the invention.

### DETAILED DESCRIPTION OF PARTICULAR EMBODIMENTS

In a preferred embodiment of the second aspect of the invention the N-amino-N'-pentylguanidine salt is the N-amino-N'-pentylguanidine hydroiodide.

In another preferred embodiment of the second aspect of the invention, the polar medium is water, acetone or a mixture thereof. Preferably the polar medium is acetone.

In a preferred embodiment of the third aspect of the invention, the polar medium is water.

In another embodiment of the third aspect of the invention the process further comprises a step iii) wherein the tegaserod free base is converted into a pharmaceutically acceptable salt. Preferably, the tegaserod free base is converted into tegaserod maleate.

The following examples are provided by way of illustration, and are not intended to be limiting of the present invention.

### EXAMPLES

### Example 1 - Preparation of APG-Hox

APG (1.23 g, 8.54 mmol) was dissolved in acetone (5 ml) at room temperature, and oxalic acid (0.769 g, 8.54 mmol) were added. Being filtered, washed with acetone and dried, 1.99 g of APG-Hox (8.49 mmol, 99.5% yield) as a white solid were obtained, with a melting point of 115-120 °C.

### Example 2 - Preparation of tegaserod free base

To a solution of APG-Hox (1.99 g, 8.49 mmol) in 26 ml H₂O, 5-MICHO (0.748 g, 4.27 mmol) was added. The resulting mixture was heated to reflux for two hours and then cooled to room temperature. 20 ml of ethyl acetate were added to extract the product. The organic phase was separated, washed with water, dried on anhydrous Na₂SO₄ and evaporated to dryness to give 2.32 g of crude tegaserod free base (90.7% yield).

The HPLC purity of the free base obtained using the oxalate salt resulted to be 99.5%

## Claims

1. An oxalate addition salt of N-amino-N'-pentylguanidine.

2. The oxalate addition salt as claimed in claim 1, wherein the molar ratio between the oxalate and the N-amino-N'-pentylguanidine is 1:1.

3. A process for preparing an oxalate addition salt as claimed in any one of the claims 1-2 which comprises the steps of:
a) reacting the N-amino-N'-pentylguanidine, as a free base or in a salt form, with oxalic acid in a polar medium; and
b) recovering the product in a solid form.

4. The process as claimed in claim 3 wherein the N-amino-N'-pentylguanidine salt is the N-amino-N'-pentylguanidine hydroiodide.

5. The process as claimed in any one of the claims 3-4, wherein the polar medium is water, acetone or a mixture thereof.

6. The process as claimed in claim 5, wherein the polar solvent is acetone.

7. A process for preparing tegaserod free base which comprises the steps of:
i) condensing the oxalate addition salt of N-amino-N'-pentylguanidine with 5-Methoxy-1 H-indole-3-carbaldehyde in a polar medium; and
ii) recovering tegaserod as free base.

8. The process as claimed in claim 6 wherein the polar medium is water.

9. The process as claimed in any one of the claims 7-8, which further comprises a step iii) wherein the tegaserod free base is converted into a pharmaceutically acceptable salt.

10. The process as claimed in claim 8 wherein the tegaserod free base is converted into tegaserod maleate.

11. Use of the oxalate addition salt of N-amino-N'-pentylguanidine as claimed in claim 1 for preparing tegaserod as a free base or in a salt form.
